# EUROPEAN PATENT APPLICATION

(11) **EP 2 399 918 A1**
(43) Date of publication of application: **28.12.2011**
(21) Application number: 10743884.8
(22) Date of filing: 19.02.2010
(51) Int. Cl.: C07D 471/22, B01J 31/22, B01J 37/08, B01J 37/34, C07D 487/22, C07D 498/22, C08K 3/04, C08K 5/3467, C08L 101/00, H01M 4/90

(54) **RING COMPOUND**

(30) Priority: 23.02.2009 JP 2009038937; 23.06.2009 JP 2009148555
(71) Applicant: Sumitomo Chemical Company, Limited, Tokyo 104-8260 (JP)
(72) Inventor: IWATA, Masatoshi, Tsukuba-shi Ibaraki 305-0821 (JP); MATSUNAGA, Tadafumi, Ibaraki-shi Osaka 567-0826 (JP); KOSHINO, Nobuyoshi, Tsukuba-shi Ibaraki 305-0821 (JP); HIGASHIMURA, Hideyuki, Tsukuba-shi Ibaraki 305-0045 (JP)
(74) Representative: Kling, Simone
(86) International application number: PCT/JP2010/053026
(87) International publication number: WO 2010/095765

(57) **Abstract**

A compound represented by the following formula (1): 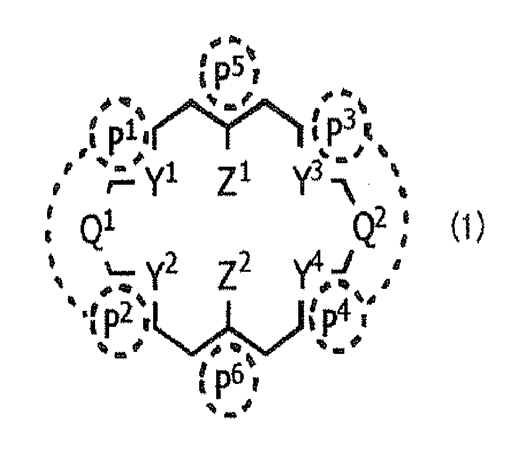 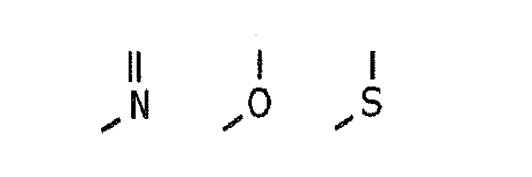 wherein, Y¹, Y², Y³ and Y⁴ represent a group represented by any of the above formulae; P¹, P², P³ and P⁴ are an atomic group required for forming an aromatic heterocyclic ring; P⁵ and P⁶ are an atomic group required for forming an aromatic ring; Z¹ and Z² represent OR^{α}, -SR^{α} or -NR^{α}₂, wherein R^{α} represents a hydrogen atom or an alkyl group having 1 to 4 carbon atoms; Q¹ and Q² represent a direct bond or a linking group; a P¹-containing ring and a P²-containing ring are bonded to form Q¹-containing fused structure; a P³-containing ring and a P⁴-containing ring are bonded to form Q²-containing fused structure; can be used as a ligand of a metal complex, and the metal complex is useful for a catalyst.

## Description

### Technical Field

The present invention relates to a ring compound, a metal complex having the same as a ligand, and a modified metal complex thereof.

### Background Art

A metal complex acts as a catalyst in a redox reaction (redox catalyst) involving electron transfer such as an oxygenation reaction, an oxidative coupling reaction, a dehydrogenation reaction, a hydrogenation reaction, an oxide decomposition reaction, or an electrode reaction, and are each used in preparation of low- and high-molecular-weight compounds. Further, it has been recently used as a phosphorescence emitting complex of organic EL materials, in addition to additives, cells, and sensor materials.

Among these metal complexes, a metal complex which has a ring compound as a ligand is stable and suppresses disassociation of metal ions, compared with a metal complex which has a non-ring compound as a ligand. Further, it is known that a metal complex having a plurality of transition metal atoms at the center thereof has an excellent activity as a redox catalyst. For example, a metal complex which has as a ligand a Schiff base type ring compound having a plurality of transition metal atoms at the center thereof has been proposed (Angew. Chem. Int. Ed., 2003, 42, 6008).

### Disclosure of Invention

However, the metal complex described above has a Schiff base type ring compound as a ligand, and therefore has an insufficient stability. Accordingly, an improvement has been demanded.

The invention provides a metal complex having an excellent stability and a useful compound as a ligand thereof.

In the first embodiment, the invention provides a compound represented by formula (1): 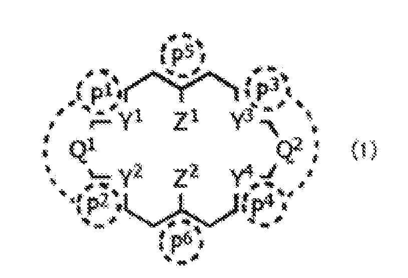 wherein,
Y¹, Y², Y³ and Y⁴ are the same as or different from each other, and represent a group represented by any one of the following formulae: 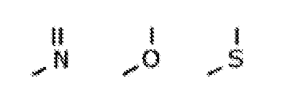
P¹ represents a group of atoms necessary for forming an aromatic heterocyclic ring together with Y¹ and the two carbon atoms adjacent to Y¹;
P² represents a group of atoms necessary for forming an aromatic heterocyclic ring together with Y² and the two carbon atoms adjacent to Y²;
P³ represents a group of atoms necessary for forming an aromatic heterocyclic ring together with Y³ and the two carbon atoms adjacent to Y³;
P⁴ represents a group of atoms necessary for forming an aromatic heterocyclic ring together with Y⁴ and the two carbon atoms adjacent to Y⁴;
P⁵ represents a group of atoms necessary for forming an aromatic ring together with the carbon atom to which Z¹ bonds and the two carbon atoms adjacent to the carbon atom to which Z¹ bonds;
P⁶ represents a group of atoms necessary for forming an aromatic ring together with the carbon atom to which Z² bonds and the two carbon atoms adjacent to the carbon atom to which Z² bonds;
   Z¹ and Z² are the same as or different from each other, and represent OR^{α}, -SR^{α} or -NR^{α}₂: wherein
   R^{α} represents a hydrogen atom or an alkyl group having 1 to 4 carbon atoms and a plurality of R^{α}'s may be the same as or different from each other:
Q¹ and Q² are the same as or different from each other, and represent a direct bond or a linking group;
a P¹-containing ring and a P²-containing ring are bonded to form Q¹-containing fused structure;
a P³-containing ring and a P⁴-containing ring are bonded to form Q²-containing fused structure;
at least one selected from a group consisting of a P¹-containing ring and a P⁵-containing ring, a P²-containing ring and a P⁶-containing ring, a P³-containing ring and a P⁵-containing ring, and a P⁴-containing ring and a P⁶-containing ring may be bonded to each other.

In the second aspect, the invention provides a metal complex which is formed of a metal atom and a ligand, wherein the ligand is the compound.

In the third aspect, the invention provides a modified metal complex which is obtained by modifying the above-described metal complex by heating, radiation, or discharge treatments until a mass reduction rate becomes 1 mass % or more and 90 mass % or less while the metal complex holds a carbon content of 5 mass % or more.

In the fourth aspect, the invention provides a modified metal complex which is obtained by modifying a mixture of the above-described metal complex, and a carbon carrier, an organic compound having a boiling point 200°C or more, or an organic compound having a thermal polymerization initiation temperature of 250°C or less by heating, radiation, or discharge treatments until a mass reduction rate becomes 1 mass % or more and 90 mass % or less while the metal complex holds a carbon content of 5 mass % or more.

In the fifth aspect, the invention provides a composition which contains the metal complex or modified metal complex and the carbon carrier or polymer.

Further, the invention provides a use of the metal complex or modified metal complex as a catalyst, particularly as an electrode catalyst for a fuel cell.

### Best Mode for Carrying out the Invention

A compound represented by formula (1) of the present invention will be described.

In formula (1), Y¹, Y², Y³ and Y⁴ are preferably -N= from the viewpoint of interaction with metal atoms during complex forming.

In formula (1), examples of an aromatic heterocyclic ring which is formed of atomic groups represented by P¹, P², P³ and P⁴ include rings such as pyridine, pyrazine, pyrimidine, furan, thiophene, thiazole, imidazole, oxazole, benzoimidazole, benzofuran, benzothiophene, isoquinoline; preferably rings such as pyridine, pyrazine, pyrimidine, furan, thiophene, thiazole, imidazole, and oxazole; and more preferably rings such as pyridine, thiazole, imidazole and oxazole.

In formula (1), examples of an aromatic ring which is formed of atomic groups represented by P⁵ and P⁶ are represented by formulae (1-a), (1-b), (1-c), (1-d) and (1-e), if they include groups corresponding to Z¹ and Z². More preferable examples include formulae (1-a), (1-b) and (1-c), and more preferably formulae (1-a) and (1-b).

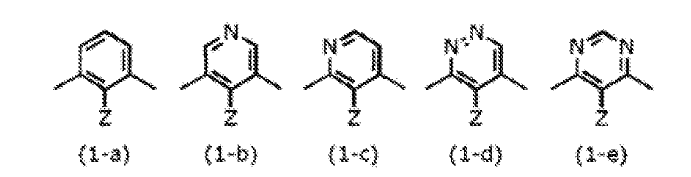

In the formulae, Z is the same as Z¹ and Z², and represents -OR^{α}, -SR^{α} or -NR^{α}₂. R^{α} represents a hydrogen atom or an alkyl group having 1 to 4 carbon atoms. A plurality of R^{α}'s may be the same as or different from each other.

In formula (1), Z¹ and Z² are preferably -OR^{α}, -SR^{α}, more preferably -OR^{α}, from the viewpoint of reaction control during synthesis.

An aromatic heterocyclic ring represented by P¹ P², P³ and P⁴ and an aromatic ring represented by P⁵ and P⁶ may have a substituent. Examples of the substituent include a halogen atom such as a fluorine, a chlorine, a bromine and an iodine; a hydroxy; a carboxyl; a mercapto; a sulfo; a nitro; a phospho; a tri(alkyl having 1 to 4 carbon atoms)silyl group; a straight, branched, or cyclic alkyl group having 1 to 50 carbon atoms such as a methyl, an ethyl, a propyl, an isopropyl, a cyclopropyl, a butyl, an isobutyl, a tert-butyl, a pentyl, a cyclopentyl, a hexyl, a cyclohexyl, a norbornyl, a nonyl, a cyclononyl, a decyl, a 3,7-dimethyloctyl, an adamantyl, a dodecyl, a cyclododecyl, a pentadecyl, an octadecyl and a docosyl; a straight, branched, or cyclic alkoxy having 1 to 50 carbon atoms such as a methoxy, an ethoxy, a propyloxy, a butoxy, a pentyloxy, a cyclohexyloxy, a norbornyloxy, a decyloxy and a dodecyloxy; an aromatic having 6 to 60 carbon atoms such as a phenyl, a 4-bromophenyl, a 2,6-dimethylphenyl, a 4-biphenyl, a 2-methylphenyl, a 3-ethenylphenyl, a pentafluorophenyl, a 4-trifluoromethylphenyl, a 3,5-dibromophenyl, a 3,5-dimethoxyphenyl, a 3,5-dihydroxyphenyl, a 4-tert-butyl-2,6-methoxymethylphenyl, a 4-tert-butylphenyl, a 4-octylphenyl, a 4-dodecylphenyl, a 4-methylphenyl, a 1-naphthyl, a 2-naphthyl and a 9-anthryl. Preferable substituents include a halogen atom such as a fluorine, a chlorine, a bromine and an iodine; a mercapto; a hydroxy; a carboxyl; an alkyl group having 1 to 20 carbon atoms such as a methyl, an ethyl, a propyl, an isopropyl, a butyl, a pentyl, a tert-butyl, a cyclohexyl, a norbornyl and an adamantyl; a straight or branched alkoxy having 1 to 10 carbon atoms such as a methoxy, an ethoxy, a propyloxy, a butoxy and a pentyloxy; an aryl having 6 to 30 carbon atoms such as a phenyl, a 4-bromophenyl, a 2,6-dimethylphenyl, a 4-biphenyl, a 2-methylphenyl, a 3-ethenylphenyl, a pentafluorophenyl, a 4-trifluoromethylphenyl, a 3,5-dibromophenyl, a 3,5-dimethoxyphenyl, a 3,5-dihydroxyphenyl, a 4-tert-butyl-2,6-methoxymethylphenyl, a 4-tert-butylphenyl, a 4-octylphenyl, a 4-dodecylphenyl, a 1-naphthyl, a 2-naphthyl and a 9-anthryl. More preferable examples include a fluorine, a bromine, a hydroxy, a carboxyl, a methyl, an ethyl, a tert-butyl, a cyclohexyl, a norbornyl, an adamantyl, a methoxy, an ethoxy, a phenyl, a 4-bromophenyl, a 2,6-dimethylphenyl, a 4-biphenyl, a 2-methylphenyl, a 3-ethenylphenyl, a pentafluorophenyl, a 4-trifluoromethylphenyl, a 3,5-dibromophenyl, a 3,5-dimethoxyphenyl, a 3,5-dihydroxyphenyl, a 4-tert-butyl-2,6-methoxymethylphenyl, a 4-tert-butylphenyl, a 4-octylphenyl, a 4-dodecylphenyl, a 2-naphthyl and a 9-anthryl. Unless otherwise specified, examples of "substituent" in the specification are the same as described above.

In formula (1), examples of Q¹-containing fused structure which is formed of P¹-containing ring and P²-containing ring, Q²-containing fused structure which is formed of P³-containing ring and P⁴-containing ring include structures represented by formulae (2-a) to (2-n); preferably structures represented by formulae (2-a), (2-g) to (2-k); and more preferably structures represented by formulae (2-a), (2-i) to (2-k). Further, these structures may have a substituent.

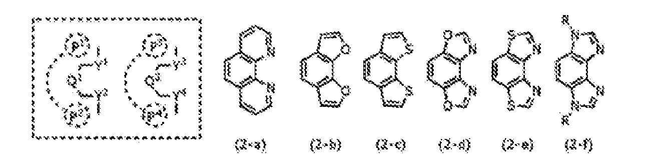

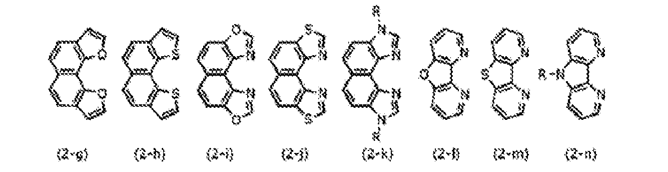

(In formulae (2-a) to (2-n), R represents a hydrogen atom or a substituent.)

Examples of the compound represented by formula (1) are preferably compounds represented by formula (2) or (3) from the viewpoint of complex forming with metal atoms. With consideration for ease of synthesis, a compound represented by the following formula (2) is more preferable. With consideration for stability of a metal complex, a compound represented by the following formula (3) is more preferable.

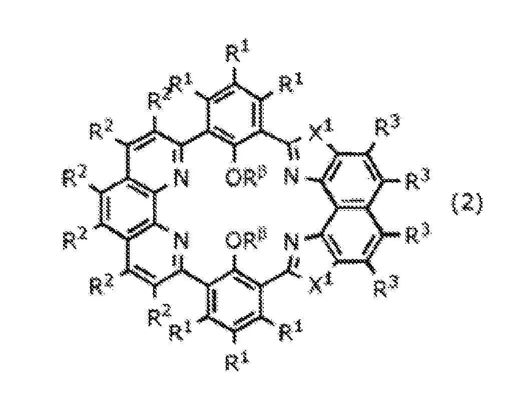

(in formula (2), R¹, R² and R³ are the same as or different from each other, and represent a hydrogen atom or a substituent. A plurality of R¹'s, R²'s and R³'s each may be the same as or different from each other. At least two of a plurality of R¹'s, R²'s and R³'s may be bonded to each other. X¹ represents -O-, -S- or -N(R^{A})- (herein, R^{A} represents a hydrogen atom or a substituent).

Two X¹' s may be the same as or different from each other. R^{β} represents a hydrogen atom or an alkyl group having 1 to 4 carbon atoms. Two R^{β}'s may be the same as or different from each other.)

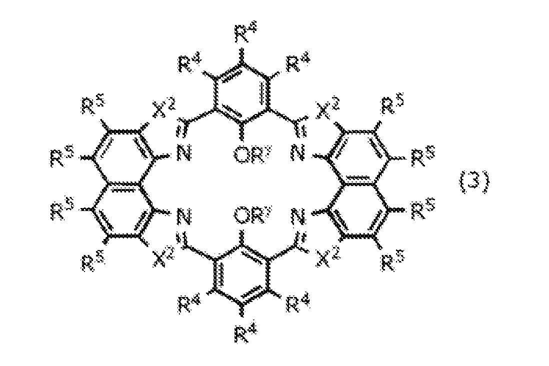

(In formula (3), R⁴ and R⁵ are the same as or different from each other, and represent a hydrogen atom or a substituent. A plurality of R⁴'s and R⁵'s each may be the same as or different from each other. At least two of a plurality of R⁴'s and R⁵'s may be bonded to each other. X² represents -O-, -S- or -N(R^{B})- (wherein R^{B} represents a hydrogen atom or a substituent). Four X²'s may be the same as or different from each other. R^{γ} represents a hydrogen atom or an alkyl group having 1 to 4 carbon atoms. Two R^{γ}'s may be the same as or different from each other.)

X¹ in formula (2) and X² in formula (3) are preferably -O-.

Examples of the compound represented by formula (2) include compounds illustrated below.

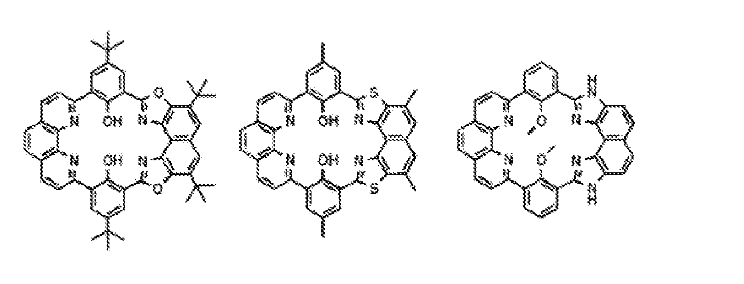

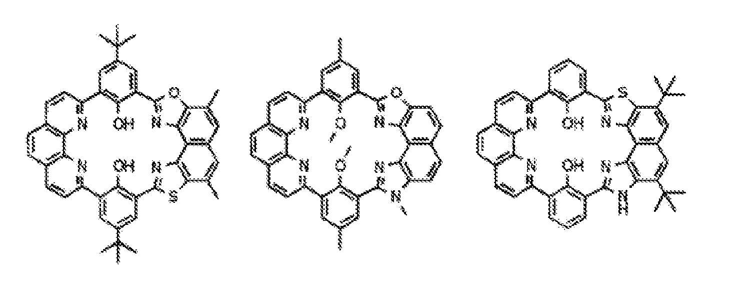

Examples of the compound represented by formula (3) include compounds illustrated below.

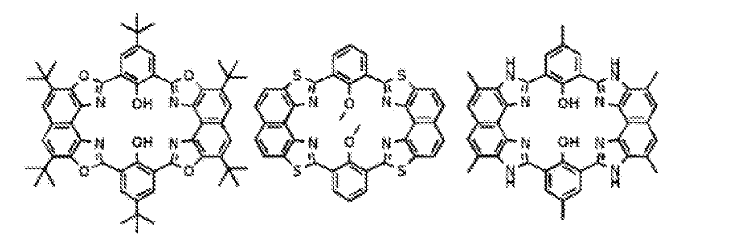

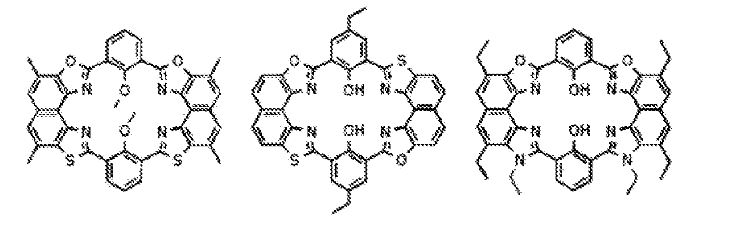

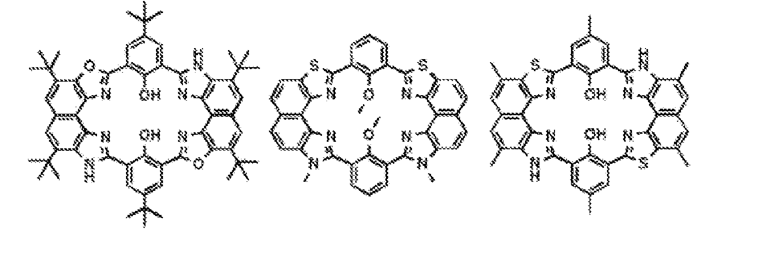

Subsequently, synthesis of the compound of the present invention will be described with compounds represented by formulae (2) and (3) that are preferable embodiments as an example.

The compound represented by formula (2) can be synthesized by condensation and dehydrogenation of a compound obtained by addition and oxidation of an organic metal reagent to a heteroring compound, followed by halogenation, cross coupling using a transition metal catalyst and formylation, as described in Tetrahedron, 1999, 55, 8377-8384; and a compound obtained by demethylation and reduction as described in Polyhedron, 2005, 24, 2618-2624.

More preferably, the compound represented by formula (2) can be synthesized by condensation and dehydrogenation of a compound represented by the following formula (4) and a compound represented by the following formula (5).

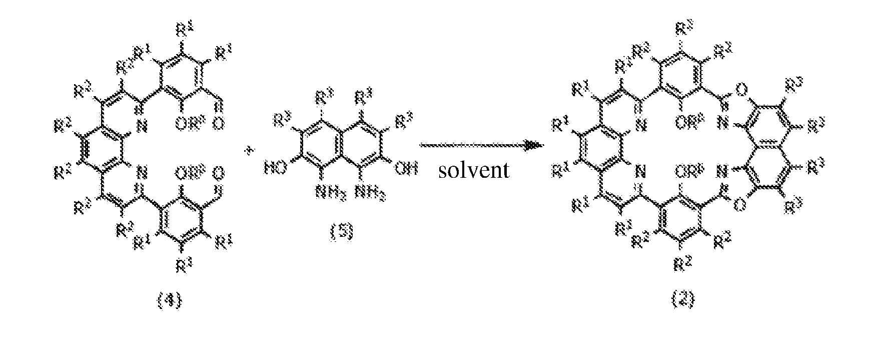

(In formulae (4) to (6), R¹, R², R³ and R^{β} are the same as described above.)

A compound represented by formula (3) can be synthesized by condensation and dehydrogenation of a 2,6-diformylphenol derivative represented by the following formula (6), and a 1,2-diaminobenzene derivative represented by the following formula (7) or a 1,8-diaminonaphthalene derivative represented by the following formula (8).

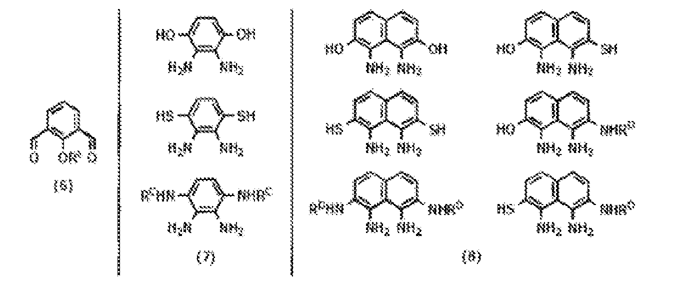

(In formulae (6) to (8), R^{δ} represents a hydrogen atom or an alkyl group having 1 to 4 carbon atoms. R^{C} and R^{D} are the same as or different from each other, and represent a hydrogen atom or a substituent).

Synthesis of compounds represented by formulae (2) and (3) preferably uses a compound containing typical metals. Examples of the typical metals are as follows. Examples of the compound containing typical metals are preferably a compound containing typical metals of Groups 13 or 14 from the viewpoint of ease of purification of product obtained after the synthesis, a compound containing tin or lead is preferable, and a compound containing tin is more preferable from the viewpoint of ease of handling.

Examples of the compound containing tin are preferably tin (II) acetate, tin (IV) acetate, tin (II) chloride, tin (IV) chloride, and more preferably tin (II) acetate, tin (IV) acetate, and tin (II) chloride.

The condensation and dehydrogenation reactions are preferably carried out by dissolving a raw material into a suitable solvent. Examples of the solvents include toluene, xylene, methanol, ethanol, isopropyl alcohol, dichloromethane, chloroform and mixtures of these solvents.

The condensation reaction can be carried out by heating in the absence of a catalyst. When a catalyst is used, the condensation can be efficiently carried out. Examples of the catalyst include acids such as p-toluenesulfonic acid, trifluoroacetic acid, trifluoromethanesulfonic acid, and phosphoric acid.

The dehydrogenation reaction can be carried out using an oxidant. Examples of the oxidant include oxygen, hydrogen peroxide, iodine, lead tetraacetate, 2,3-dichloro-5,6-dicyano-1,4-benzoquinone, chloranil, chromium (VI) oxide, osmium tetraoxide, potassium permanganate, manganese (IV) oxide, iodobenzene diacetate, [bis(trifluoroacetoxy)iodo]benzene, palladium or platinum supported on activated carbon or silica gel.

A reaction temperature for condensation and dehydrogenation reactions is usually 0 to 300°C, preferably 0 to 250°C, and more preferably 0 to 200°C. A reaction time for condensation and dehydrogenation is usually 1 minute to 1 week, preferably 5 minutes to 100 hours, and more preferably 1 hour to 48 hours. Reaction temperature and reaction time are depending on the combination of acids and solvents and can be adjusted appropriately.

A polymer of the compound represented by formula (1) can be used for the following uses in the same manner as the compound represented by formula (1).

The polymer is a polymer having a residue of the compound represented by formula (1). The polymer is a polymer having a residue of the compound represented by formula (1), that is to say, a polymer having a group comprising an atomic group obtained by removing one or more, generally one, hydrogen atoms in the compound represented by formula (1). Examples of the polymer include a conductive polymer, a dendrimer, a natural polymer, a solid polyelectrolyte, polyethylene, polystyrene, polyacrylonitrile, polyethylene glycol, and polypropylene. Of those, a conductive polymer, a solid polyelectrolyte, polystyrene and polyacrylonitrile are preferable, and polystyrene and polyacrylonitrile are more preferable. The term "conductive polymer" is a collective term for polymer substances each showing metallic or semi-metallic conductivity. Examples of the conductive polymer include: polyacetylene and a derivative of polyacetylene, polyparaphenylene and a derivative of polyparaphenylene, polyparaphenylene vinylene and a derivative of polyparaphenylene vinylene, polyaniline and a derivative of polyaniline, polythiophene and a derivative of polythiophene, polypyrrole and a derivative of polypyrrole, polyfluorene and a derivative of polyfluorene, polyfluorene and a derivative of polyfluorene, polycarbazole and a derivative of polycarbazole, and polyindole and a derivative of polyindole described in "Conductive Polymer" (written by Shinichi Yoshimura, KYORITSU SHUPPAN CO., LTD) and "New Applications of Conducting Polymers" (edited by Yukio Kobayashi, CMC Publishing CO., LTD.); and copolymers of the conductive polymers. Examples of the solid polymer electrolyte include polymers obtained by sulfonating perfluorosulfonic acid, polyether ether ketone, polyimide, polyphenylene, polyarylene, and polyarylene ether sulfone.

The compound of the present invention can be used as compounds for the ligand of a metal complex. In other words, the metal complex of the present invention is formed of the compound of the present invention and a metal atom. When two metal atoms are present, the metal atoms may be coordinated with each other by bridge coordination. The metal atom contains a transition metal atom and typical metal atom. The transition metal means an element of Groups 3 to 12 in periodical table, and the typical metal means a metal element other than the transition metal. The transition metal atom and typical metal atom may be an uncharged or a charged ion.

Examples of the transition metals include scandium, titanium, vanadium, chromium, manganese, iron, cobalt, nickel, copper, zinc, yttrium, zirconium, niobium, molybdenum, technetium, ruthenium, rhodium, palladium, silver, cadmium, hafnium, tantalum, tungsten, rhenium, osmium, iridium, platinum, gold, and mercury.

Examples of the typical metal include lithium, sodium, potassium, rubidium, cesium, beryllium, magnesium, calcium, strontium, barium, aluminum, gallium, indium, thallium, tin, lead, and bismuth.

Examples of the metal atom are preferably a transition metal atom of fourth to sixth Period of the Periodic Table from a practical point of view, more preferably, titanium, vanadium, manganese, iron, cobalt, nickel, copper, zinc, niobium, molybdenum, ruthenium, rhodium, palladium, silver, tantalum, tungsten, rhenium, osmium, iridium, platinum, and gold; further more preferably titanium, vanadium, manganese, iron, cobalt, nickel, copper, zinc, molybdenum, ruthenium, rhodium, palladium, silver, tungsten, iridium, platinum atoms; and most preferably manganese, iron, cobalt, nickel, copper, and zinc atoms.

The metal complex of the present invention may include a neutral molecule, or a counter ion capable of electrically neutralizing the metal complex. Examples of the neutral molecule include a molecule that solvates to form a solvated salt and a compound to be a ligand other than compounds represented by formula (1). Examples of the neutral molecule include water, methanol, ethanol, propanol, isopropyl alcohol, 2-methoxyethanol, 1,1-dimethylethanol, ethylene glycol, N,N'-dimethylformamide, N,N'-dimethylacetamide, N-methyl-2-pyrrolidone, dimethylsulfoxide, acetone, chloroform, acetonitrile, benzonitrile, triethylamine, pyridine, pyrazine, diazabicyclo[2,2,2]octane, 4,4'-bipyridine, tetrahydrofuran, diethyl ether, dimethoxyethane, methyl ethyl ether, 1,4-dioxane, acetic acid, propionic acid, 2-ethyl hexanoic acid; and preferably water, methanol, ethanol, isopropyl alcohol, ethylene glycol, N,N'-dimethylformamide, N,N'-dimethylacetamide, N-methyl-2-pyrrolidone, chloroform, acetonitrile, benzonitrile, triethylamine, pyridine, pyrazine, diazabicyclo[2,2,2]octane, 4,4'-bipyridine, tetrahydrofuran, dimethoxyethane, 1,4-dioxane, acetic acid, propionic acid, and 2-ethylhexanoic acid.

As the counter ion, because the transition metal atom and typical metal atom generally have a positive charge, a negative ion which neutralizes the atom electrically is selected. Examples of the counter ion include a fluoride ion, a chloride ion, a bromide ion, an iodide ion, a sulfide ion, an oxide ion, a hydroxide ion, a hydride ion, a sulfite ion, a phosphate ion, a cyanide ion, an acetate ion, a 2-ethylhexanoate ion, a carbonate ion, a sulfate ion, a nitrate ion, a hydrogen carbonate ion, a trifluoroacetate ion, a thiocyanide ion, a trifluoromethane sulfonate ion, an acetylacetonate, a tetrafuloroborate ion, a hexafluorophosphate ion, and a tetraphenylborate ion, and preferably a chloride ion, a bromide ion, an iodide ion, an oxide ion, a hydroxide ion, a hydride ion, a phosphate ion, a cyanide ion, an acetate ion, a 2-ethylhexanoate ion, a carbonate ion, a sulfate ion, a nitrate ion, an acetylacetonate, and a tetraphenylborate ion. In addition, when a plurality of X's are present, the X's may be the same as or different from each other, or a neutral molecule and an ion may be coexistent with each other.

Examples of the metal complex of the present invention include the following metal complexes.

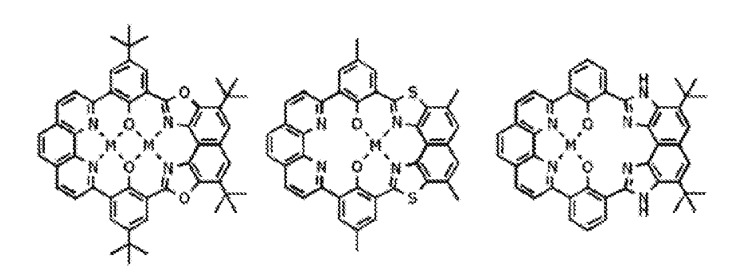

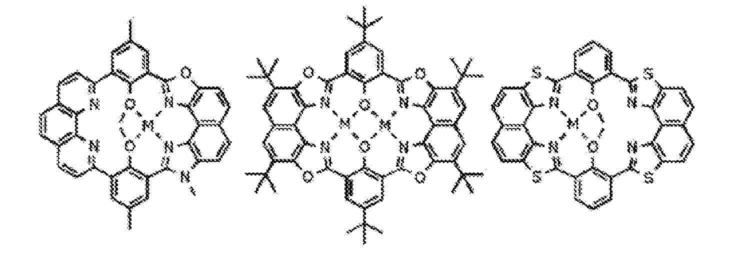

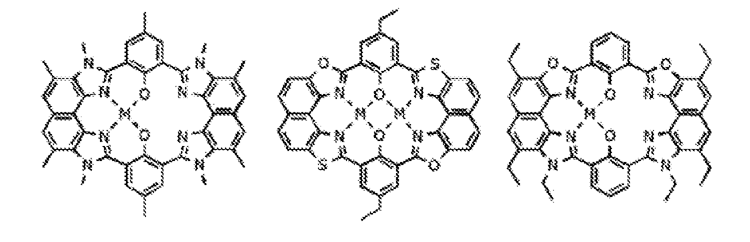

(In the formula, M represents a metal atom. The metal atom represented by M is the same as the description in the metal atom described above. Two M's may be the same as or different from each other.)

Next, a method of synthesizing the metal complex of the present invention will be explained.

The metal complex of the present invention, for example, can be prepared by mixing the compound of the present invention and a reagent (hereinafter, referred to as "metal reagent") as a metal atom source. The metal reagent is a compound having a metal atom and general examples are metal salts.

The metal complex of the present invention can be obtained by mixing the compound represented by formula (1) and a metal reagent in an appropriate reaction solvent.

Examples of the reaction solvent include water, acetic acid, aqueous ammonia, methanol, ethanol, propanol, isopropyl alcohol, 2-methoxyethanol, 1-butanol, 1,1-dimethyl ethanol, ethylene glycol, diethyl ether, 1,2-dimethoxyethane, methyl ethyl ether, 1,4-dioxane, tetrahydrofuran, benzene, toluene, xylene, mesitylene, durene, decalin, dichloromethane, chloroform, carbon tetrachloride, chlorobenzene, 1,2-dichlorobenzene, N,N'-dimethylformamide, N,N'-dimethylacetamide, N-methyl-2-pyrrolidone, dimethylsulfoxide, acetone, acetonitrile, benzonitrile, triethylamine, pyridine, pyrazine, diazabicyclo[2.2.2]octane and mixture of these solvents, preferably one capable of dissolving a compound represented by formula (1) and a metal reagent.

A reaction temperature is generally -10 to 250°C, preferably 0 to 200°C, and more preferably 0 to 150°C.

A reaction time is generally 1 minute to 1 week, preferably 5 minutes to 24 hours, and more preferably 1 hour to 12 hours. Reaction temperature and reaction time can be optimized depending on the kinds of the compound represented by formula (1) and the metal reagent. As a method involving isolating the produced metal complex from the reaction solution after the reaction and purifying the metal complex, an optimum method selected from a known recrystallization method, a known redeposit method, and a known chromatography method can be appropriately employed, and two or more of these methods may be employed in combination. The produced metal complex may precipitate depending on the kind of the reaction solvent. The precipitated metal complex can be isolated and purified by separating the metal complex by a separation method such as filtration and subjecting the separated product to a washing operation and a drying operation as required.

Since the basic structures of the metal complexes having the compound represented by the above formula (1) as a ligand are constituted by aromatic heterocyclic rings and aromatic rings and each of the complexes has high heat resistance and has an excellent stability even at a high temperature, and hence has a high catalytic activity.

The metal complexes are particularly suitable for use as, for example, redox catalysts, and specific examples of the applications of the metal complexes include: decomposition catalysts for hydrogen peroxide; oxidation polymerization catalysts for aromatic compounds; catalysts for purifying an exhaust gas and waste water; redox catalyst layers for dye sensitization solar cells; carbon dioxide reduction catalysts; catalysts for the production of reformed hydrogen; and oxygen sensors.

Further, since each of the metal complexes has an expanded conjugation, the metal complexes can be used as organic semiconductor materials such as organic EL materials, organic transistors and dye sensitized solar cells.

The metal complex of the present invention can be used as it is, but may be subject to a modification treatment to be used as a modified metal complex. The modification treatment is carried out by modifying the metal complex of the present invention by heating, radiation or discharge treatments, until a mass reduction rate (reduction rate of mass from before treatment to after treatment) becomes 1 mass % or more and 90 mass % or less while the metal complex holds a carbon content of 5 mass % or more. The thus obtained modified metal complex is a metal complex with increased stability. The metal complex to be used for the modification treatment may be one metal complex or two or more metal complexes.

As pretreatment for the modification treatment, the metal complex is particularly preferable to be dried at a temperature of 15°C or higher or 200°C or lower under reduced pressure of 1333 Pa or lower for 6 hours or longer. The pretreatment may be carried out using a vacuum drier or the like.

The treatment of the metal complex is preferably carried out in the presence of hydrogen, helium, nitrogen, ammonia, oxygen, neon, argon, krypton, xenon, acetonitrile, or a gas mixture of these gases. It is preferably in the presence of hydrogen, helium, nitrogen, ammonia, oxygen, neon, argon, or a gas mixture of these gases; and more preferably in the presence of hydrogen, nitrogen, ammonia, argon, or a gas mixture of these gases. Further, pressure during the modification treatment may be varied depending on an optional modification treatment.

Heating treatment means heating a metal complex. The temperature at which the metal complex is subjected to a heating treatment is not particularly limited as long as the mass reduction rate becomes 1 mass % or more and 90 mass % or less. The temperature for the heating treatment is preferably 200°C or higher, for example 200 to 1200°C; more preferably 300°C or higher. In addition, an upper limit of the temperature for heating treatment is not particularly limited as long as the carbon content of the modified product after the treatment (the carbon content means a content rate of carbon atoms which can be determined, for example, by elemental analysis) is 5 mass % or more; the temperature is preferably 1,200°C or lower, more preferably 1,000°C or lower, and further preferably 800°C or lower.

The treatment time for the heating treatment may be set properly depending on the gas to be used, temperature and the like.

In the state that the gas is tightly closed or ventilated, the temperature may be gradually increased from room temperature, (1) to an aimed temperature and then decreased immediately or (2) to keep the temperature after the temperature reached the aimed temperature and the metal complex can be gradually treated. From the viewpoint of durability, (2) is preferable. The time period for which the temperature is held at the aimed temperature is not particularly limited as long as the time period is preferably 1 to 100 hours, more preferably 1 to 40 hours, still more preferably 2 hours to 10 hours, or particularly preferably 2 to 3 hours.

As apparatus for the heating treatment, an oven, a furnace, an IH hot plate, and the like can be used.

Examples of the modification treatments for substituting the heating treatment include radiation irradiation treatment and discharge treatment.

Radiation irradiation treatment means irradiating the metal complex of the present invention with radioactive rays selected from electromagnetic waves (α-rays, (3-rays, neutron rays, electron rays, 7-ray, X-rays, vacuum ultraviolet rays, ultraviolet rays, visible rays, infrared rays, microwaves, radio waves, a laser) and particle beams.

The radiation irradiation treatment preferably means irradiating the metal complex of the present invention with radioactive rays selected from X-rays, electron rays, ultraviolet rays, visible rays, infrared rays, microwaves, and a laser, more preferably radioactive rays selected from electron rays, visible rays, infrared rays, microwaves, and a laser.

Discharge treatment means carrying out a discharge selected from corona, glow, and plasma discharges (including low temperature plasma discharge) to the metal complex of the present invention. The discharge treatment is preferably a low temperature plasma discharge.

Radiation irradiation treatment and discharge treatment may be carried out according to instruments and treatment methods to be used generally for surface reforming treatment of polymer films and for example, methods described in a literature (Adhesion Society of Japan, "Chemistry of Surface Analysis, Reformation", issued by Nikkan Kogyo Shimbun on 2003), etc. can be employed.

Treatment time, of radiation irradiation treatment and discharge treatments, is preferably within 10 hours, more preferably within 3 hours, even more preferably within 1 hour and particularly preferably within 30 minutes.

It is preferable that heating, radiation, or discharge treatments are carried out until the metal complex has a mass reduction rate of 2 to 90 mass %, but too low a mass reduction rate tends to pose a difficulty in maintaining a complex structure, more preferably 2 to 80 mass %, and particularly preferably 3 to 70 mass %.

Further, the carbon content in the modified metal complex may be held at 5 mass % or more, but from the viewpoint of the degree of assemblage of the metal atom in the modified metal complex, 10 mass % or more, more preferably 20 mass % or more, even more preferably 30 mass % or more, and particularly preferably 40 mass % or more. Therefore, it is preferable to avoid such conditions as heating in the oxygen atmosphere for long period of time so as to reduce the carbon content.

The modified metal complex of the present invention can be obtained by modifying a mixture (hereinafter, referred to as "metal complex mixture") of a metal complex and a carbon carrier, an organic compound having a boiling point of 200°C or more, or an organic compound having a thermal polymerization initiating temperature of 250°C or less by heating, radiation, or discharge treatments until a mass reduction rate (a reduction rate of a mixture from before treatment to after treatment) becomes 1 mass % or more to 90 mass % or less while the metal complex holds a carbon content of 5 mass % or more.

The amount of the metal complex in the metal complex mixture is preferably 1 mass % or more to 70 mass % or less, more preferably 2 mass % or more to 60 mass % or less, and particularly preferably 3 mass % or more to 50 mass % or less. Further, the total content of the metal complex in a metal complex mixture of a carbon carrier, an organic compound having a boiling point of 200°C or more, or an organic compound having a thermal polymerization initiation temperature of 250°C or less is generally 30 mass % or more to 99 mass % or less, and preferably 50 mass % or more to 90 mass % or less.

Examples of the carbon carrier include carbon particles such as Norit, Ketjen black, Vulcan, black pearl, acetylene black; fullerene such as C60 and C70; carbon nanotubes, carbon nanohorns, carbon fibers and the like.

Examples of the organic compound having a boiling point or melting point of 200[deg.] C. or more are aromatic carboxylic acid derivatives such as perylene-3,4,9,10-tetracarboxylic dianhydride, 3,4,9,10-perylenetetracarboxylic acid diimide, 1,4,5,8-naphthalenetetracarboxylic dianhydride, 1,4,5,8-naphthalenetetracarboxylic acid diimide, 1,4,5,8-naphthalenetetracarboxylic acid, pyromellitic acid, and pyromellitic dianhydride. The structures of these compounds are shown below.

A value for which "calc" is appended to a boiling point (b.p.) shown in the following structural formulae is a calculated value of a boiling point registered in SciFinder (version 2007.2) which is a software provided from Chemical Abstract Service, and other values are measured values. Further, melting points are described for the purpose of reference.

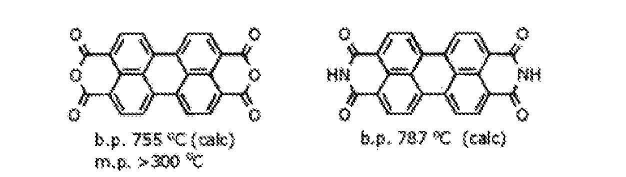

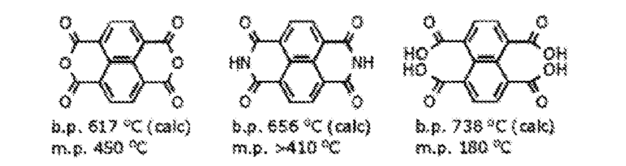

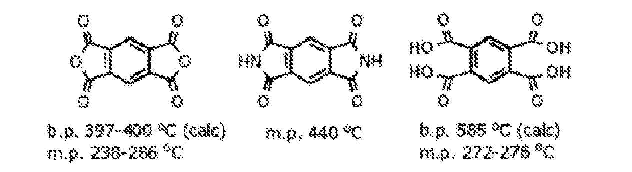

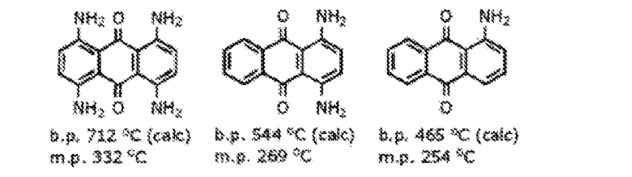

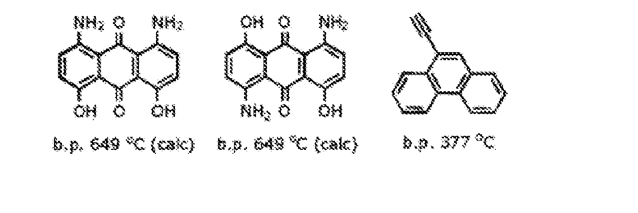

The organic compound having a thermal polymerization initiation temperature of 250°C or less is an organic compound having an aromatic ring and an unsaturated bond (double bond, triple bond), and organic compounds such as acenaphthylene and vinylnaphthalene represented by the following formula. The temperature shown below the compounds is a measured value of a polymerization initiation temperature for each compound. A five-membered ring of acenaphthylene is not an aromatic ring and has a double bond which is an unsaturated bond in a ring.

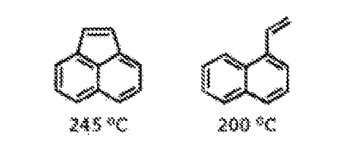

Conditions for modifying the metal complex mixture by heating, radiation irradiation, or discharge treatments are the same as that for modifying only a metal complex by heating, radiation irradiation, or discharge treatments. Further, in the metal complex mixture, a metal complex, a carbon carrier, an organic compound having a boiling point of 200°C or more, or an organic compound having a thermal polymerization initiation temperature of 250°C or less can be used alone or in combination with two or more.

Further, the metal complex and the modified metal complex of the present invention may be used alone, or may be used as a composition in combination with a carbon carrier, polymer or mixture of them from the viewpoint of stability, catalyst activity of the metal complex and modified metal complex.

Further, the metal complex and modified metal complex of the present invention may be used alone or in combination with other compounds as catalysts such as fuel cell electrode catalysts.

The composition of the present invention contains a metal complex or a modified metal complex and a carbon carrier or polymer. The amount of the metal complex or modified metal complex in the composition is usually 1 mass % or more to 70 mass % or less, preferably 3 mass % or more to 50 mass % or less; the total amount of the carbon carrier or polymer is generally 30 mass % or more to 99 mass % or less, and preferably 50 mass % or more to 90 mass % or less.

The carbon carrier in the composition of the present invention is the same as described above.

Examples of the polymer include polyethylene, polypropylene, polyacrylonitrile, polyester, polyacetylene, polyaniline, polypyrrole, and polythiophene.

In the composition of the present invention, a metal complex, a modified metal complex, a carbon carrier and a polymer can be used alone and in combination with two or more.

The metal complex, modified metal complex, and composition of the present invention may be used as a fuel cell electrode catalyst or film deterioration inhibitor, an aromatic compound oxidative coupling catalyst, a waste gas and water purification catalyst, an oxidation-reduction catalyst layer for a dye sensitized solar cell, a carbon dioxide reduction catalyst, a reformed hydrogen-producing catalyst, an oxygen sensor as well as organic semiconductor materials such as organic electroluminescence materials, organic transistors and dye sensitized solar cells, and a peroxide decomposition catalyst such as hydrogen peroxide decomposition catalyst. When the metal complex, modified metal complex, and composition may be used as a hydrogen peroxide decomposition catalyst, they suppress generation of hydroxy radicals, while they can decompose into water and oxygen.

Further, the metal complex, modified metal complex and composition of the present invention are useful as an oxidative coupling catalyst of an aromatic compound. In this case, in producing polymers such as polyphenylene ether and polycarbonate, they can be used as an oxidative coupling catalyst.

Further, the metal complex, modified metal complex and composition of the present invention are useful as a hydrodesulfurization and denitrification catalyst for transforming a sulfur oxide or nitrogen oxide in an exhaust gas from factories and automobiles into hydrogen sulfide, sulfuric acid or ammonia.

In addition, the metal complex, modified metal complex and composition of the present invention are useful as a catalyst for modifying carbon monoxide in reformed hydrogen. The reformed hydrogen contains CO, so there is a case where a fuel electrode is poisoned due to carbon monoxide when the reformed hydrogen is used in a fuel cell. The catalyst can be used to reduce the concentration of the carbon monoxide.

### Examples

Hereinafter, the invention will be described in detail based on Examples.

### Example 1 (Synthesis of Ring compound (Ala))

A ring compound (A1a) was synthesized according to the following reaction formula.

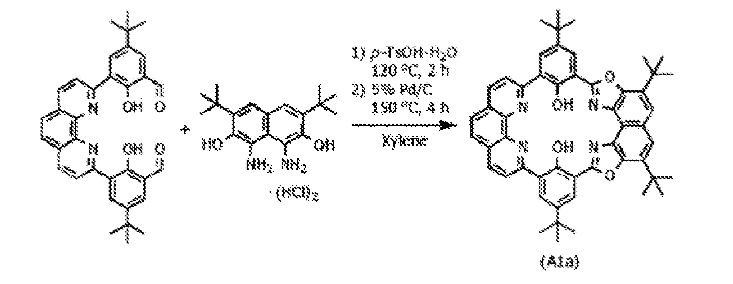

Aldehyde compound as a starting material was prepared according to the description in Tetrahedron, 1999, 55, 8377-8384. 1,8-diamino-2,7-dihydroxynaphthalene was obtained as a dihydrochloride by preparing 1,8-dinitro-2,7-dimethoxynaphthalene according to the description of Polyhedron, 2005, 24, 2618-2624, followed by demethylation of a methoxy group and reduction of a nitro group. Specifically it is as follows.

Under a nitrogen atmosphere, to 5 mL of solution of 20mg of aldehyde compound and 20mg of 1,8-diamino-2,7-dihydroxynaphthalene dihydrochloride in xylene was added 5mg of p-toluenesulfonic acid monohydrate to form a mixture, the mixture was stirred at 120°C for 2 hours, followed by cooling to room temperature. To the resultant solution was added 50 mg of 5 wt % Pd/C at room temperature, followed by stirring at 150°C for 4 hours. The resultant solution was cooled to room temperature, insoluble matter was removed by filtration, further volatile components were evaporated using an evaporator to recover a greenish brown residue to obtain a ring compound (A1a).
¹H NMR (CDCl₃, 300MHz, δ): 1.50 (s, 18H, ^{t}Bu), 1.63 (s, 18H, ^{t}Bu), 7.72, 8.07, 8.10, 8.43, 8.92, 9.13 (2H, ArH). APPI-MS: 795.3 ([M+H]⁺)

### (Synthesis of Metal Complex (B1a))

A metal complex (B1a) was synthesized according to the following reaction formula. 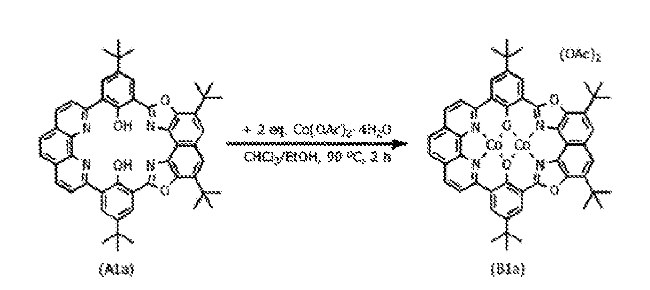 (in the formula, OAc represents acetate ion, and hereinafter has the same meaning)

Under a nitrogen atmosphere, to 38mg of ring compound (A1a) and 25 mg of cobalt acetate tetrahydrate was added 10 mL of chloroform/ethanol (1:1 (volume ratio)) mixed solvent. The resultant solution was stirred at 90°C for 2 hours to precipitate a yellow powder. The yellow powder was filtered and dried to obtain a metal complex (B1a).
ESI-MS: 969.2 ([M-OAc]⁺), 455.1 ([M-20Ac]²⁺).

### Example 2 (Synthesis of Metal Complex (B2a))

A metal complex (B2a) was synthesized according to the following reaction formula.

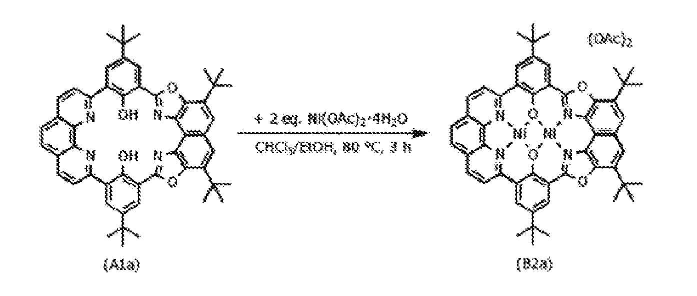

Under a nitrogen atmosphere, to 20mg of ring compound (A1a) and 13 mg of nickel acetate tetrahydrate was added 6 mL of chloroform/ethanol (1:1 (volume ratio)) mixed solvent. The resultant solution was stirred at 80°C for 3 hours, and then volatile components were evaporated by an evaporator to obtain a brown powder. The brown powder was washed with diethyl ether and dried to obtain a metal complex (B2a). ESI-MS: 969.1 ([M-OAc]⁺).

### Example 3 (Synthesis of Metal Complex (B3a))

A metal complex (B3a) was synthesized according to the following reaction formula.

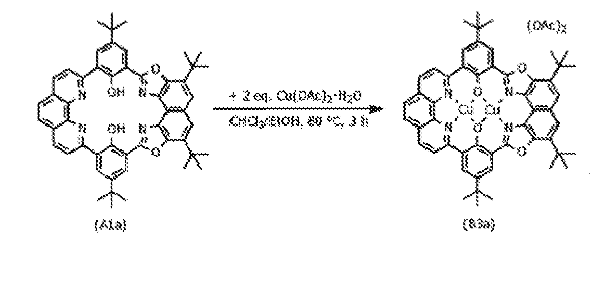

Under a nitrogen atmosphere, to 20mg of ring compound (A1a) and 10 mg of copper acetate monohydrate was added 6 mL of chloroform/ethanol (1:1 (volume ratio)) mixed solvent. The resultant solution was stirred at 80°C for 3 hours, and then volatile components were evaporated by an evaporator to obtain a brown powder. The brown powder was washed with diethyl ether and dried to obtain a metal complex (B3a). ESI-MS: 979.1 ([M-OAc]⁺), 460.1 ([M-20Ac]²⁺).

### Example 4 (Synthesis of Metal Complex (B4a))

A metal complex (B4a) was synthesized according to the following reaction formula.

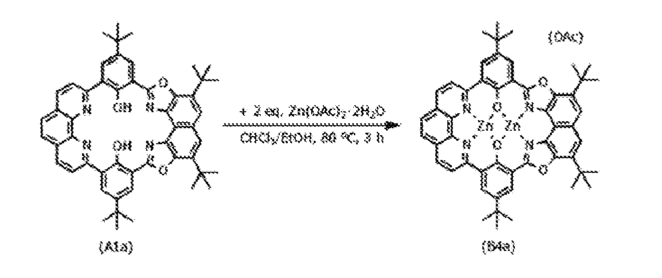

Under a nitrogen atmosphere, to 10mg of ring compound (A1a) and 8 mg of zinc acetate dihydrate was added 2 mL of chloroform/ethanol (1:1 (volume ratio)) mixed solvent. The resultant solution was stirred at 80°C for 3 hours to precipitate a yellow powder. The yellow powder was filtered off and dried to obtain a metal complex (B4a). ESI-MS: 983.2 ([M-OAc]⁺).

### Example 5 (Synthesis of Ring compound (A1b))

A ring compound (A1b) was synthesized according to the following reaction formula.

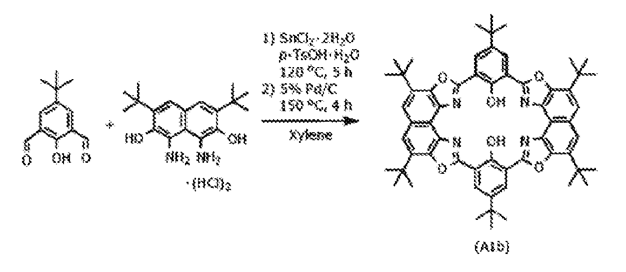

Under a nitrogen atmosphere, 10 mL of a mixed solution of 40 mg of 1,8-diamino-2,7-dihydroxynaphthalene dihydrochloride and 20mg of aldehyde compound in xylene was prepared in a 100 mL eggplant flask, 5 mg of p-toluenesulfonic acid monohydrate and 4mg of tin (II) chloride dihydrate was added thereto, followed by stirring at 120°C for 5 hours. Subsequently, heating was stopped, cooling by leaving to stand was carried out, then 10 mg of 5 wt % Pd/C was added thereto followed by stirring at 150°C for 5 hours. The resultant solution was cooled to room temperature, and then insoluble matter was removed by filtration, and further volatile components were evaporated by an evaporator. The resultant residue was purified by a silica gel column to yield a ring compound (A1b) as a brown solid.
ESI-MS: 937.4 ([M+H]⁺).

### (Synthesis of Metal Complex (B1b))

A metal complex (B1b) was synthesized according to the following reaction formula.

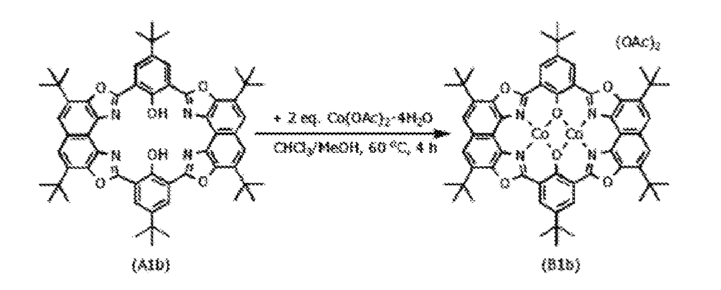

Under a nitrogen atmosphere, to 7 mg of ring compound (A1b) and 4mg of cobalt acetate tetrahydrate was added 10 mL of chloroform/methanol (1:1 (volume ratio)) mixed solvent in a 50 mL eggplant flask. The resultant mixture was stirred at 60°C for 4 hours, and volatile components were evaporated by an evaporator to obtain a metal complex (B1b).
ESI-MS: 1111.2 ([M-OAc]⁺).

### Example 6

Metal complex (B1a) and a carbon carrier (trade name: Ketjen Black 600JD, high density: 15 to 50kg/m³, manufactured by Lion Corporation) were mixed at a weight ratio of 1:4. The resultant mixture was stirred in methanol at room temperature, and dried at a reduced pressure of 200 Pa for 12 hours to obtain a metal complex mixture (C1a).

The metal complex compound (C1a) was modified by being raised to 600°C at a rate of 200°C/hour and then subsequently heated at 600°C for 2 hours in a tubular furnace (program-controllable opening and closing type tubular furnace, trade name: EPKRO-14R, manufactured by Isuzu Seisakusho) under a nitrogen atmosphere (nitrogen gas flow of 200 mL/min), to obtain a modified metal complex (D1a).

### Example 7

A corresponding metal complex mixture (C1b) and a corresponding modified metal complex (D1b1) were obtained in the same manner as Example 6 except that a metal complex (B1a) was replaced with a metal complex (B1b) in Example 6.

### Example 8

A corresponding modified metal complex (D1b2) was obtained in the same manner as Example 7 except that the heating temperature of metal complex mixture (C1b) was changed from 600 to 800°C in Example 7.

### Comparative Example 1 (Synthesis Of Metal Complex (B2))

A metal complex (B2) was synthesized according to the following reaction formula.

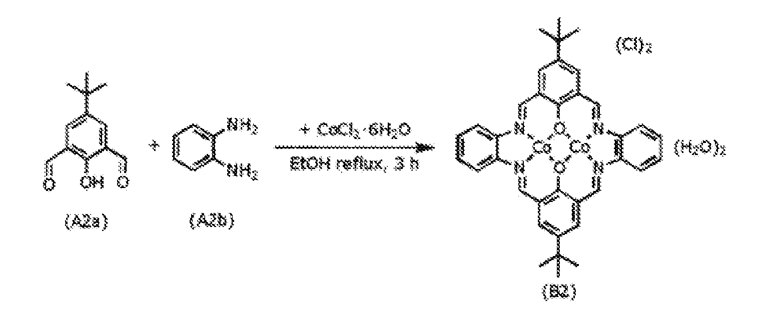

Under a nitrogen atmosphere, 5 mL of a solution containing 0.238g of cobalt chloride hexahydrate and 0.192g of 4-t-butyl-2,6-diformylphenol (A2a) in ethanol was prepared, followed by stirring at room temperature. To the ethanol solution was gradually added 10 mL of a solution containing 0.108g of 1,2-diaminobenzene (A2b) in ethanol. The resultant mixture was refluxed for 3 hours to precipitate a ginger powder. The ginger powder was filtered and dried to obtain a metal complex (B2).
Analyzed calculated value of C₃₆H₃₄C₁₂Co₂N₄O₂·2H₂O: C, 55.47; H, 4.91; N, 7.19 measured value: C, 56.34; H, 4.83; N, 7.23

### Comparative Example 2

Metal complex (B2) and a carbon carrier (trade name: Ketjen Black 300J, high density: 100 to 145kg/m³, manufactured by Lion Corporation) were mixed at a weight ratio of 1:4. The resultant mixture was stirred in methanol at room temperature, and dried at a reduced pressure of 200 Pa for 12 hours to obtain a metal complex mixture (C2).

The metal complex mixture (C2) was modified by being raised to 600°C at rate of 200°C/hour and then subsequently heated at 600°C for 2 hours in a tubular furnace (program-controllable opening and closing type tubular furnace, trade name: EPKRO-14R, manufactured by Isuzu Seisakusho) under a nitrogen atmosphere (nitrogen gas flow of 200 mL/min), to obtain a modified metal complex (D2).

### Oxygen Reduction Reaction Measurement

First, 2 mg of modified metal complex mixture (C1a) was charged into a sample bottle, 0.6 mL of water, 0.4 mL of ethanol, and 20 µL of Nafion (registered trademark) solution (manufactured by Aldrich, 5 wt % solution) were added thereto, and ultrasonic waves were irradiated for 30 minutes. 10.00 µL of the resultant suspension was dropped onto a disk portion of a ring disk electrode (manufactured by Nikko-Keisoku, trade name: NRDE-4, disk portion: glassy carbon (diameter 6.0mm), ring portion: platinum (ring inner diameter 7.0 mm, ring outer diameter 9.0 mm)) and dried at room temperature overnight to produce a measuring electrode.

Then, the measuring electrode was rotated at 600 rpm by a rotating ring disk electrode device (manufactured from Nikko-Keisoku, trade name: RRDE-1) while current density values under an oxygen atmosphere and nitrogen atmosphere were measured respectively at room temperature. Measurement of current density was carried out by scanning at a scan rate of 5 mV/s from 0.725 V to -0.225V using 0.05 mol/L aqueous sulfuric solution (25°C) as a cell solution, a silver/silver chloride electrode (saturated potassium chloride solution) as a reference electrode, and a platinum electrode (wire shape) as a counter electrode, using a dual electrochemical analyzer (manufactured by ALS, trade name: ALS model 701C).

A value which subtracts current density measured under nitrogen atmosphere from current density measured under oxygen atmosphere was set as a current density of oxygen reduction reaction, and a current density of metal complex mixture (C1a) at 0.3V (vs RHE) potential under oxygen atmosphere was set as i_{C1a}.

A series of operations described above were carried out with respect to metal complex mixtures (C1b), (C2), and modified metal complexes (D1a), (D1b1), (D2). Current density values of metal complex mixtures (C1b), (C2), modified metal complexes (D1a), (D1b1), and (D2) at 0.3V (vs RHE) potential under oxygen atmosphere were set as i_{C1b}, i_{C2}, i_{D1a}, i_{D1b1}, i_{D2} respectively.

From the obtained measured values, values obtained by dividing current density values of oxygen reduction reaction after heating by current density values before heating were calculated as current density ratios before and after heating (i_{D1a}/i_{C1a}, i_{D1b1}/i_{C1b}, i_{D2}/i_{C2}), and are shown in Table 1

**[Table 1]**

| | | Current density ratio before and after heating |
|---|---|---|
| Example 6 | i_{D1a}/i_{C1a} | 2.72 |
| Example 7 | i_{D1b1}/i_{C1b} | 8.41 |
| Comparative Example 2 | i_{D2}/i_{C2} | 1.60 |

As can be understood from Table 1, since metal complexes produced in Examples 6 and 7 had current density ratios before and after heating of 2.72 and, 8.41, it is considered that they had stable complex structures as modified metal complexes even after heating, in comparison with a metal complex produced in Comparative Example 2 where current density ratio before and after heating did not exceed 1.60. Therefore, it is considered that the metal complex of the present invention has excellent stability. As a result, it is considered that the metal complex and modified metal complex of the present invention have an excellent oxygen reductive capacity.

### Industrial Applicability

The metal complex of the present invention has an excellent stability, particularly thermal stability. Further, the compound as the ligand of the present invention is a ring compound which forms a Schiff base site as a cyclic structure, and is useful in synthesis of the metal complex. The modified metal complex of the present invention has an oxygen reductive capacity with high activity, and therefore can provide a highly applicable catalyst. Further, in a preferable embodiment of the present invention, the modified metal complex of the present invention has high hydrogen oxidation capability.

## Claims

1. A compound represented by the following formula (1): 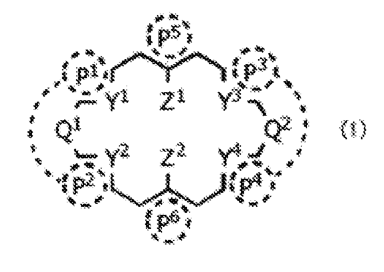 wherein,
Y¹, Y², Y³ and Y⁴ are the same as or different from each other, and represent a group represented by any one of the following formulae: 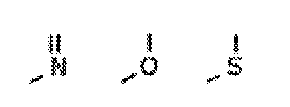
P¹ represents a group of atoms necessary for forming an aromatic heterocyclic ring together with Y¹ and the two carbon atoms adjacent to Y¹;
P² represents a group of atoms necessary for forming an aromatic heterocyclic ring together with Y² and the two carbon atoms adjacent to Y²;
P³ represents a group of atoms necessary for forming an aromatic heterocyclic ring together with Y³ and the two carbon atoms adjacent to Y³;
P⁴ represents a group of atoms necessary for forming an aromatic heterocyclic ring together with Y⁴ and the two carbon atoms adjacent to Y⁴;
P⁵ represents a group of atoms necessary for forming an aromatic ring together with the carbon atom to which Z¹ bonds and the two carbon atoms adjacent to the carbon atom to which Z¹ bonds;
P⁶ represents a group of atoms necessary for forming an aromatic ring together with the carbon atom to which Z² bonds and the two carbon atoms adjacent to the carbon atom to which Z² bonds;
Z¹ and Z² are the same as or different from each other, and represent OR^{α}, -SR^{α} or -NR^{α}₂: wherein
R^{α} represents a hydrogen atom or an alkyl group having 1 to 4 carbon atoms and a plurality of R^{α}'s may be the same as or different from each other:
Q¹ and Q² are the same as or different from each other, and represent a direct bond or a linking group;
a P¹-containing ring and a P²-containing ring are bonded to form Q¹-containing fused structure;
a P³-containing ring and a P⁴-containing ring are bonded to form Q²-containing fused structure;
at least one selected from a group consisting of a P¹-containing ring and a P⁵-containing ring, a P²-containing ring and a P⁶-containing ring, a P³-containing ring and a P⁵-containing ring, and a P⁴-containing ring and a P⁶-containing ring may be bonded to each other.

2. The compound according to Claim 1, wherein the compound represented by formula (1) is a compound represented by formula (2) or (3): 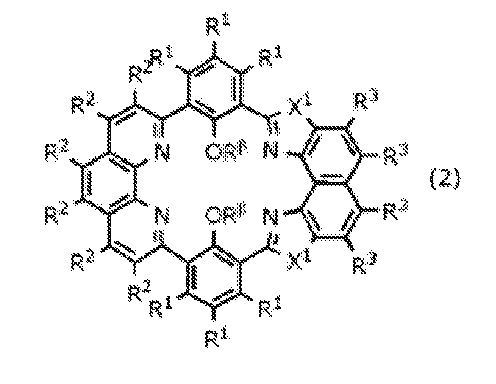 wherein,
R¹, R² and R³ are the same as or different from each other, and represent a hydrogen atom or a substituent;
a plurality of R¹'s, R²'s and R³'s each may be the same as or different from each other;
at least two of a plurality of R¹'_{,}s, R²'s and R³'s may be bonded to each other;
X¹ represents -O-, -S- or -N(R^{A})-:
wherein
R^{A} represents a hydrogen atom or a substituent:
two X¹'s may be the same as or different from each other;
R^{β} represents a hydrogen atom or an alkyl group having 1 to 4 carbon atoms;
and two R^{β}'s may be the same as or different from each other: or 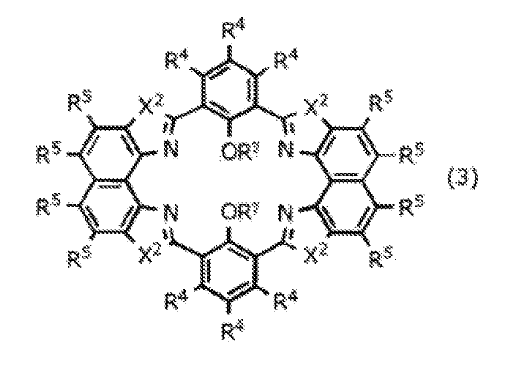 wherein
R⁴ and R⁵ are the same as or different from each other, and represent a hydrogen atom or a substituent;
a plurality of R⁴'s and R⁵'s each may be the same as or different from each other;
at least two of a plurality of R⁴'s and R⁵'s may be bonded to each other;
X² represents -O-, -S- or -N(R^{B})-:
wherein
R^{B} represents a hydrogen atom or a substituent:
four X²'s may be the same as or different from each other;
R^{γ} represents a hydrogen atom or an alkyl group having 1 to 4 carbon atoms; and
two R^{γ}'s may be the same as or different from each other.

3. The compound according to Claim 2, wherein the substituent is a halogen atom; a hydroxy group; a carboxyl group; a mercapto group; a sulfo group; a nitro group; a phospho group; a tri(alkyl group having 1 to 4 carbon atoms)silyl group; a straight, branched or cyclic alkyl group having 1 to 50 carbon atoms; a straight, branched or cyclic alkoxy group having 1 to 50 carbon atoms or an aromatic group having 6 to 60 carbon atoms.

4. The compound according to Claim 1, wherein the compound represented by formula (1) is a compound represented by the following formula (3): 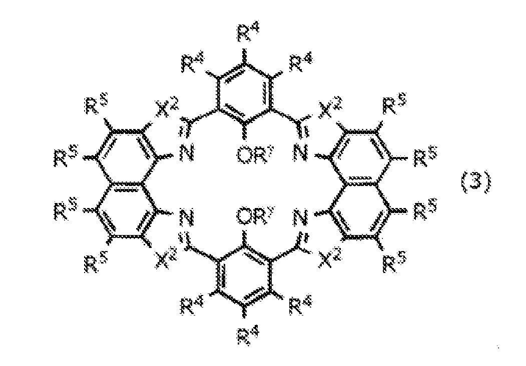 wherein
R⁴ and R⁵ are the same as or different from each other, and represent a hydrogen atom or a substituent;
a plurality of R⁴'s and R⁵'s each may be the same as or different from each other;
at least two of a plurality of R⁴'s and R⁵'s may be bonded to each other;
X² represents -O-, -S- or -N(R^{B})-:
wherein
R^{B} represents a hydrogen atom or a substituent:
four X²'s may be the same as or different from each other;
R^{γ} represents a hydrogen atom or an alkyl group having 1 to 4 carbon atoms; and
two R^{γ}'s may be the same as or different from each other.

5. The compound according to Claim 4, wherein the substituent is a halogen atom; a hydroxy group; a carboxyl group; a mercapto group; a sulfo group; a nitro group; a phospho group; a tri(alkyl group having 1 to 4 carbon atoms)silyl group; a straight, branched or cyclic alkyl group having 1 to 50 carbon atoms; a straight, branched or cyclic alkoxy group having 1 to 50 carbon atoms or an aromatic group having 6 to 60 carbon atoms.

6. The compound according to Claim 4,
wherein
X² is -O- and
the substituent is a halogen atom; a hydroxy group; a carboxyl group; a mercapto group; a sulfo group; a nitro group; a phospho group; a tri(alkyl group having 1 to 4 carbon atoms)silyl group; a straight, branched or cyclic alkyl group having 1 to 50 carbon atoms; a straight, branched or cyclic alkoxy group having 1 to 50 carbon atoms or an aromatic group having 6 to 60 carbon atoms.

7. The compound according to Claim 1, represented by any one of the following formulae:

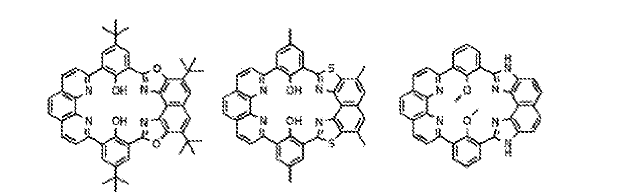

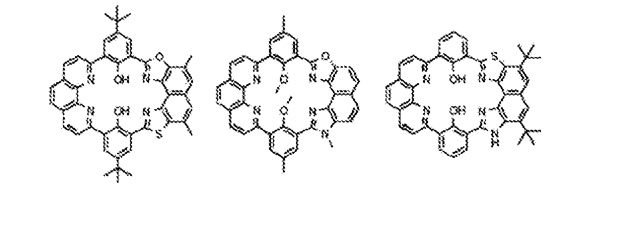

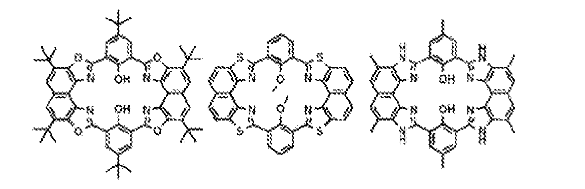

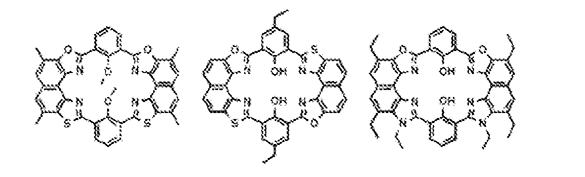

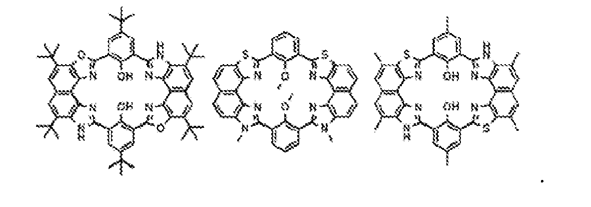

8. A metal complex which is formed of a metal atom and a ligand, wherein the ligand is the compound according to any one of Claims 1 to 7.

9. The metal complex according to Claim 8, wherein the metal atom is a transition metal atom of fourth to sixth Period of the Periodic Table.

10. The metal complex according to Claim 8, represented by any one of the following formulae: 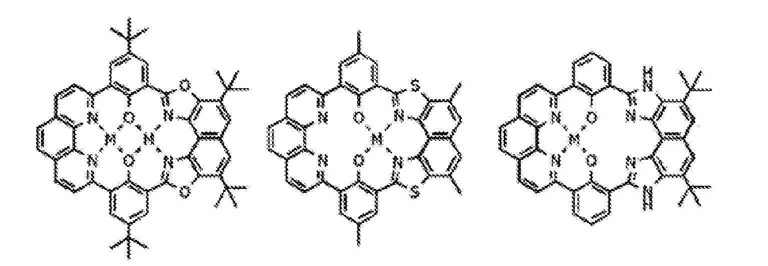 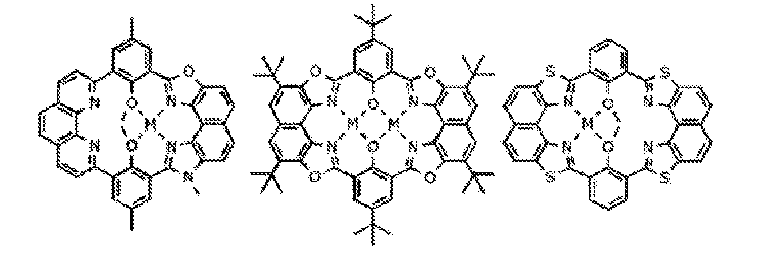 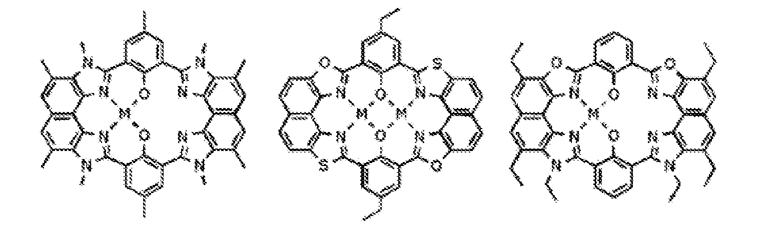 wherein M represents a metal atom; and two M's may be the same as or different from each other.

11. The metal complex according to Claim 10, wherein the metal atom is titanium, vanadium, manganese, iron, cobalt, nickel, copper, zinc, niobium, molybdenum, ruthenium, rhodium, palladium, silver, tantalum, tungsten, rhenium, osmium, iridium, platinum or gold.

12. A modified metal complex which is obtained by modifying the metal complex according to Claim 8 by heating, radiation, or discharge treatments until a mass reduction rate becomes 1 mass % or more and 90 mass % or less while the metal complex holds a carbon content of 5 mass % or more.

13. A modified metal complex which is obtained by modifying a mixture of the metal complex according to Claim 8, a carbon carrier, and an organic compound having a boiling point of 200°C or more or an organic compound having a thermal polymerization initiation temperature of 250°C or less, by heating, radiation, or discharge treatments until a mass reduction rate becomes 1 mass % or more and 90 mass % or less while the metal complex holds a carbon content of 5 mass % or more.

14. The modified metal complex according to Claim 13, which is obtained by modifying a mixture of the metal complex according to Claim 8, a carbon carrier, and an organic compound having a boiling point of 200°C or more or an organic compound having a thermal polymerization initiation temperature of 250°C or less, by heating at 200 to 1200°C until a mass reduction rate becomes 1 mass % or more to 90 mass % or less while the metal complex holds a carbon content of 5 mass % or more.

15. A composition comprising the metal complex according to Claim 8, and a carbon carrier or a polymer.

16. A composition comprising the modified metal complex according to claim 12, and a carbon carrier or a polymer.

17. Use of the metal complex according to Claim 8 as an electrode catalyst for a fuel cell.

18. Use of the modified metal complex according to Claim 12 or 13 as an electrode catalyst for a fuel cell.
